# EUROPEAN PATENT APPLICATION

(11) **EP 3 437 550 A1**
(43) Date of publication of application: **06.02.2019**
(21) Application number: 16895810.6
(22) Date of filing: 28.03.2016
(51) Int. Cl.: A61B 5/00

(54) **HEALTH SURVEILLANCE METHOD AND HEALTH SURVEILLANCE GARMENT**

(71) Applicant: Shenzhen Royole Technologies Co., Ltd., Shenzhen, Guangdong 518052 (CN)
(72) Inventor: JIANG, Chao, Shenzhen Guangdong 518052 (CN)
(74) Representative: Hamer, Christopher K.
(86) International application number: PCT/CN2016/077555
(87) International publication number: WO 2017/166029

(57) **Abstract**

A method for health monitoring and a health monitoring garment are provided. The method for health monitoring includes: obtaining physical state information of a monitored subject via a health monitoring garment including a flexible display screen; determining whether the monitored subject needs an aid according to the physical state information; obtaining aid measure information corresponding to the physical state information according to the physical state information based on a determination that the monitored subject needs the aid; indicating the aid measure information via the flexible display screen. The method for health monitoring can improve timeliness and reliability of health monitoring.

## Description

### TECHNICAL FIELD

The present disclosure relates to the field of health monitoring technology, and particularly to a method for health monitoring and a health monitoring garment adopting the method for health monitoring.

### BACKGROUND

With the development of the mobile and Internet medical industry, the smart medical hardware based on the Internet and mobile communication emerge one after another, which has undoubtedly greatly facilitated consumers' daily health monitoring. At present, common medical smart hardware products such as smart blood glucose meters, smart blood pressure monitors, smart wristbands, smart clothes, smart backpacks, and the like can implement the monitoring of one or more physical state information and perform data exchange with mobile terminals, such as smart phones, via wireless networks, so that a user can intuitively understand his/her physical state through the mobile terminal.

Although the above-identified medical smart hardware products implement the monitoring of one or more physical state information, they can only present monitoring data to the user. However, when the physical state of the user is abnormal, they cannot provide corresponding aid measure according to abnormal physical state information. Especially in the case of health monitoring for patients suffering from sudden illness or inconvenient mobility, when the patient has a sudden illness or falls, the related medical smart hardware products cannot provide emergency aid measures to the patients or people around them against the emergency. They can only passively wait for the arrival of ambulance personnel, which may result in missing the optimal rescue time.

### SUMMARY

With regard to the problem of the related art, embodiments of the disclosure provide a method for health monitoring, so as to provide aid measures corresponding to physical state information according to the physical state information of a monitored subject, thereby improving timeliness and reliability of health monitoring and emergency aid.

According to the embodiments of the disclosure, a health monitoring garment adopting the method for health monitoring is also provided.

The method for health monitoring includes the following.

Physical state information of a monitored subject is obtained via a health monitoring garment, where the health monitoring garment includes a flexible display screen.

Whether the monitored subject needs an aid is determined according to the physical state information.

Aid measure information corresponding to the physical state information is obtained according to the physical state information based on a determination that the monitored subject needs the aid.

The aid measure information is indicated via the flexible display screen.

As one implementation, whether the monitored subject needs the aid is determined according to the physical state information as follows.

The physical state information of the monitored subject is compared with a preset state information range.

Determine that the monitored subject does not need the aid when the physical state information falls within the preset state information range.

Determine that the monitored subject needs the aid when the physical state information does not fall within the preset state information range.

As one implementation, the method further includes the following after determining whether the monitored subject needs the aid and before obtaining the aid measure information according to the physical state information.

Detect whether network communication of a monitoring server is normal.

As one implementation, when the network communication of the monitoring server is normal, the aid measure information is obtained according to the physical state information as follows.

Abnormal state information corresponding to the physical state information is generated when the physical state information does not fall within the preset state information range;

The abnormal state information is sent to the monitoring server.

The aid measure information is received from the monitoring server.

The aid measure information is obtained by the operation selected from the group consisting of generating the aid measure information by the monitoring server according to the abnormal state information, and inputting in the monitoring server the aid measure information by a monitor according to the abnormal state information received by the monitoring server.

As one implementation, the method further includes the following after sending the abnormal state information to the monitoring server.

A waiting time after sending the abnormal state information to the monitoring server is recorded.

Preset aid measure information in a memory is read according to the abnormal state information when the waiting time exceeds a preset time threshold value.

As one implementation, the method further includes the following after determining that the monitored subject needs the aid.

Abnormal state information corresponding to the physical state information is generated when the physical state information does not fall within the preset state information range.

Preset aid measure information in a memory is read according to the abnormal state information.

As one implementation, the preset aid measure information in the memory is read according to the abnormal state information as follows.

The abnormal state information is compared with multiple preset abnormal information in the memory.

Preset abnormal information that matches the abnormal state information is obtained from the multiple preset abnormal information.

The preset aid measure information in the memory is read, according to the preset abnormal information that matches the abnormal state information.

As one implementation, the method further includes the following after obtaining the aid measure information according to the physical state information.

The aid measure information is indicated in a voice broadcast manner.

As one implementation, the physical state information includes physiological state information and physical posture information of the monitored subject, where the physiological state information includes physical temperature information, heart rate information, blood glucose information, blood oxygen information, blood pressure information, electrocardiogram information, and pulse information; and the physical posture information includes moving speed information, physical tilt angle information, and physical orientation information.

The aid measure information includes a part that needs to be aided displayed on the flexible display screen.

The aid measure information includes an aid method displayed on the flexible display screen.

A health monitoring garment is provided. The garment includes a wearable body, a controller disposed on the wearable body, a physical state monitoring module, and a flexible display screen.

The controller is electrically connected with the physical state monitoring module and the flexible display screen.

The physical state monitoring module is configured to obtain physical state information of a monitored subject and to send the physical state information to the controller.

The controller is configured to receive the physical state information, to determine whether the monitored subject needs an aid according to the physical state information, and to obtain aid measure information corresponding to the physical state information according to the physical state information based on a determination that the monitored subject needs the aid.

The flexible display screen is configured to indicate the aid measure information.

The garment further includes a memory electrically connected with the controller, where the memory is configured to store a preset state information range and multiple preset aid measure information. The controller configured to determine whether the monitored subject needs the aid according to the physical state information is configured to: compare the physical state information of the monitored subject with the preset state information range, determine that the monitored subject does not need the aid when the physical state information falls within the preset state information range, and determine that the monitored subject needs the aid when the physical state information does not fall within the preset state information range.

The garment further includes a communication module configured to communicate with a monitoring server. After the controller determines whether the monitored subject needs the aid, the communication module is further configured to detect whether network communication of the monitoring server is normal.

When the network communication of the monitoring server is normal, the controller configured to obtain the aid measure information corresponding to the physical state information according to the physical state information is configured to: generate abnormal state information corresponding to the physical state information when the physical state information does not fall within the preset state information range, control the communication module to send the abnormal state information to the monitoring server, and control the communication module to receive the aid measure information from the monitoring server.

The aid measure information is obtained by the operation selected from the group consisting of generating the aid measure information by the monitoring server according to the abnormal state information, and inputting in the monitoring server the aid measure information by a monitor according to the abnormal state information received by the monitoring server.

The memory is further configured to store a preset time threshold value. After the controller controls the communication module to send the abnormal state information to the monitoring server, the controller is further configured to: record a waiting time after sending the abnormal state information to the monitoring server, and read the preset aid measure information in the memory according to the abnormal state information when the waiting time exceeds the preset time threshold value.

The controller is further configured to: generate abnormal state information corresponding to the physical state information when the physical state information does not fall within the preset state information range, and read the preset aid measure information in the memory according to the abnormal state information.

The memory is further configured to store multiple preset abnormal information corresponding to multiple aid measure information. The controller configured to read the preset aid measure information in the memory according to the abnormal state information is configured to: compare the abnormal state information with the multiple preset abnormal information in the memory, obtain, from the multiple preset abnormal information, preset abnormal information that matches the abnormal state information, and read the preset aid measure information in the memory, according to the preset abnormal information that matches the abnormal state information.

The aid measure information includes a part that needs to be aided and an aid method displayed on the flexible display screen.

The physical state monitoring module includes a physiological state monitoring sub-module and a physical posture monitoring sub-module.

The physiological state monitoring sub-module is configured to obtain physiological state information of the monitored subject.

The physical posture monitoring sub-module is configured to obtain physical posture information of the monitored subject.

The physiological state information includes physical temperature information, heart rate information, blood glucose information, blood oxygen information, blood pressure information, electrocardiogram information, and pulse information and the physical posture information includes moving speed information, physical tilt angle information, and physical orientation information.

According to the method for health monitoring, the physical state information of the monitored subject is obtained, and the aid measure information corresponding to the physical state information is obtained according to the physical state information based on a determination that the monitored subject needs the aid. After that, the part that needs to be aided of the body of the monitored subject and the aid measure information are displayed on the flexible display screen. When the monitored subject need the aid, the aid measure information can be presented to people around the monitored subject via the flexible display screen and the part that needs to be aided of the monitored subject can be displayed on the flexible display screen. In this manner, people around the monitored subject can conduct emergency aid on the monitored subject according to the aid measure information and the part that needs to be aided, thereby having higher timeliness and reliability.

### BRIEF DESCRIPTION OF THE DRAWINGS

To describe technical solutions in the embodiments of the present disclosure more clearly, the following briefly introduces the accompanying drawings required for describing the embodiments.
FIG. 1 is a schematic flow diagram illustrating a method for health monitoring according to an embodiment of the present disclosure.
FIG. 2 is another schematic flow diagram illustrating a method for health monitoring according to an embodiment of the present disclosure.
FIG. 3 is a schematic structural view illustrating a health monitoring garment according to an embodiment of the present disclosure.
FIG. 4 is another schematic structural view illustrating a health monitoring garment according to an embodiment of the present disclosure.
FIG. 5 is a schematic diagram illustrating a usage sate of a health monitoring garment according to an embodiment of the present disclosure.

### DETAILED DESCRIPTION OF ILLUSTRATED EMBODIMENTS

Technical solutions in the embodiments of the present disclosure will be described clearly and completely hereinafter with reference to the accompanying drawings in the embodiments of the present disclosure. Apparently, the described embodiments are merely some rather than all embodiments of the present disclosure. All other embodiments obtained by those of ordinary skill in the art based on the embodiments of the present disclosure without creative efforts shall fall within the protection scope of the present disclosure.

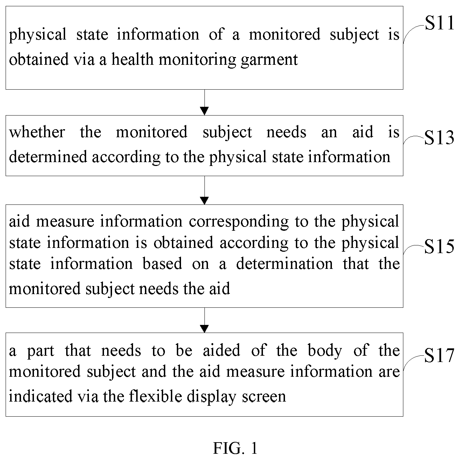

Referring to FIG. 1, as one implementation, a method for health monitoring is provided. The method is configured to monitor a physical state of a monitored subject, and to obtain and present aid measures corresponding to physical state information of the monitored subject when determining that the monitored subject needs an aid. In this way, timeliness and reliability of health monitoring and emergency aid can be improved. The method for health monitoring at least includes the following operations.

At S11, physical state information of a monitored subject is obtained via a health monitoring garment. The health monitoring garment includes a flexible display screen covering any part, where the display screen is configured to indicate a part(s) of the monitored subjects or an aid measure(s).

At S13, whether the monitored subject needs an aid is determined according to the physical state information.

At S15, aid measure information corresponding to the physical state information is obtained according to the physical state information based on a determination that the monitored subject needs the aid.

At S17, a part that needs to be aided of the body of the monitored subject and the aid measure information are indicated via the flexible display screen.

The method may be executed by smart clothing such as health monitoring garments or the like. The health monitoring garment includes a wearable body, a controller disposed on the wearable body, a physical state monitoring module, and a flexible display screen. The physical state monitoring module is configured to obtain the physical state information of the monitored subject and to send the physical state information to the controller. The controller is configured to receive the physical state information, to determine whether the monitored subject needs the aid according to the physical state information, and to obtain the aid measure information according to the physical state information based on a determination that the monitored subject needs the aid. Thereafter, the part that needs to be aided of the body of the monitored subject and the aid measure information are indicated via the flexible display screen. In the embodiment, the wearable body is in a form of a garment, which can be worn on the monitored subject and is in elastic contact with the body of the monitored subject. The flexible display screen covers an outer surface of the wearable body. When the wearable body is worn on the monitored subject, the flexible display screen can accurately indicate any part covered by the health monitoring garment of the monitored subject. In addition, the wearable body can also be a part of the garment, such as a chest, a back, a sleeve, etc., or adhered to the worn garment by means of adhesive. Furthermore, the wearable body is not limited to the tops, but may also include pants, hats, shoes, and the like.

The monitored subject can be an elderly person, a patient, or one who needs health monitoring. When the monitored subject is under health monitoring, the health monitoring garment is worn on the monitored subject. The physical state information includes but is not limited to physiological state information and physical posture information of the monitored subject, where the physiological state information includes but is not limited to physical temperature information, heart rate information, blood glucose information, blood oxygen information, blood pressure information, electrocardiogram information, and pulse information of the monitored subject, and the physical posture information includes but is not limited to moving speed information, physical tilt angle information, and physical orientation information of the monitored subject. It can be understood that, a physiological state of the monitored subject can be understood by obtaining the physiological state information of the monitored subject such as physical temperature degrees, blood glucose concentration, blood oxygen concentration, blood pressure level, electrocardiogram, pulse rate, and the like. Accordingly, whether the monitored subject needs the aid and how to aid the monitored subject are determined according to the physiological state. For example, when the blood oxygen concentration is too low, it is determined that the monitored subject is in an extreme hypoxic state; in this situation, the monitored subject needs to be aided immediately in an oxygen inhalation manner. Moreover, abnormal physical state information of the monitored subject and aid measure information corresponding to the abnormal physical state information of the monitored subject can be displayed on the flexible display screen, for example, can be displayed by text "the blood oxygen concentration of the patient is too low; please aid the patient immediately in the oxygen inhalation manner".

In addition, it is possible to understand a physical posture of the monitored subject by obtaining the physical posture information of the monitored subject such as physical moving speed, physical tilt angle, physical orientation, and the like. Accordingly, whether the monitored subject needs the aid is determined according to the posture state. For example, when the physical moving speed is zero and the physical tilt angle exceeds a certain angle, it is determined that the monitored subject may be in a sleep state or fall. The current physical orientation of the monitored subject can be obtained by obtaining the physical orientation information, for example, supine or prone. In this situation, combined with the physiological state information, whether the monitored subject needs the aid is determined. For example, when the blood glucose concentration is too low and the monitored subject is in a supine posture, it is determined that the monitored subject may be fainted due to a low blood glucose concentration; in this situation, the monitored subject needs to be aided by injecting glucose. And then, the flexible display screen can display abnormal physical state information of the monitored subject and aid measure information corresponding to the abnormal physical state information of the monitored subject thereon, for example, by displaying a text "the blood glucose concentration of the patient is too low; please aid the patient by injecting glucose". In this manner, people around the monitored subject can know the cause of syncope in time, and know emergency aid measures, thereby improving timeliness and reliability of the emergency aid.

Whether the monitored subject needs the aid is determined according to the physical state information as follows.

The physical state information of the monitored subject is compared with a preset state information range.

Determine that the monitored subject does not need the aid when the physical state information falls within the preset state information range.

Determine that the monitored subject needs the aid when the physical state information does not fall within the preset state information range.

The preset state information range refers to a physiological state information range or a physical posture information range on condition that the physical state is normal, such as physical temperature range, blood glucose concentration range, blood oxygen concentration range, blood pressure range, tilt angle range under a standing posture, etc. It is understood that, the preset state information range can be preset according to different situations of each monitored subject. When the monitored subject is subjected to health monitoring, the physical state information of the monitored subject is obtained and then compared with the preset state information range. In this manner, whether the physical state information of the monitored subject falls within a normal range can be determined. That is, determine that the monitored subject needs the aid when the physical state information of the monitored subject does not fall within the normal range.

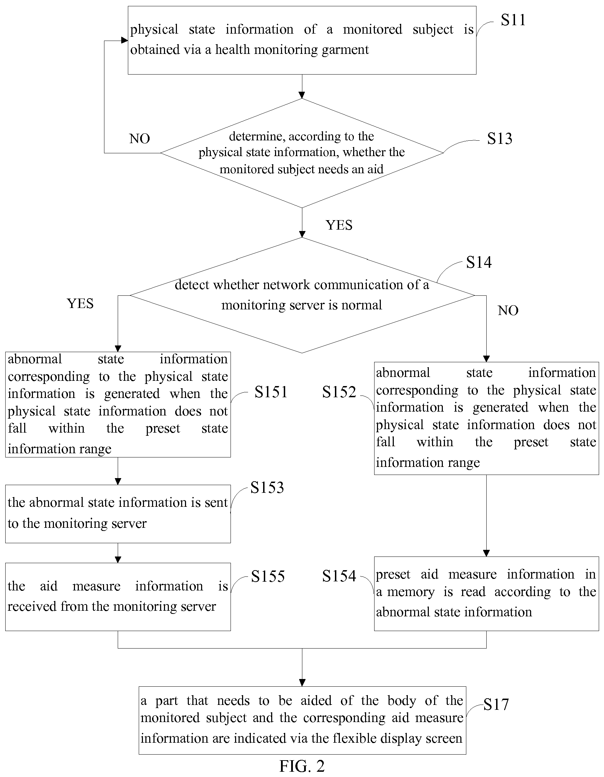

Referring to FIG. 2, the method further includes the following after determining whether the monitored subject needs the aid and before obtaining the aid measure information according to the physical state information.

At S14, detect whether network communication of a monitoring server is normal.

The health monitoring garment further includes a communication module. The communication module is configured to communicate with the monitoring server. The monitoring server can be a computer in a monitoring room of the hospital, and be attended by a dedicated monitor (such as a doctor or a nurse). The monitoring server can also be a smart phone or a tablet computer carried by the monitor. The health monitoring garment can establish a communication connection and exchange communication data with the monitoring server via the communication module. Whether network communication of the monitoring server is normal can be detected as follows. The health monitoring garment sends test data to the monitoring server and records the sending time of the test data. The health monitoring garment receives feedback data from the monitoring server. When a difference between a time of receiving the feedback data and a time of sending the test data exceeds a preset time length (e.g., 3 seconds), or the health monitoring garment still does not receive the feedback data when the sending time exceeds the preset time length, it is determined that the network communication of the monitoring server is in an abnormal state. Otherwise, after sending the test data, if the health monitoring garment can receive the feedback data in the preset time length, it is determined that the network communication of the monitoring server is normal. The feedback in response to the text data is automatically completed by the monitoring server, as long as the text data is received, the monitoring server will send the feedback data automatically without manual intervention.

In the embodiment, whether the network communication of the monitoring server is normal can be detected, so as to determine the source where the aid measure information is obtained. As a result, it is possible to avoid the situation where the aid measure information cannot be obtained normally and thus the emergency aid action cannot be implemented due to abnormal network communication of the monitoring server.

As one implementation, when the network communication of the monitoring server is normal, the aid measure information is obtained according to the physical state information as follows.

At S151, abnormal state information corresponding to the physical state information is generated when the physical state information does not fall within the preset state information range.

At S153, the abnormal state information is sent to the monitoring server.

At S155, the aid measure information is received from the monitoring server.

The aid measure information is obtained by the operation selected from the group consisting of generating the aid measure information by the monitoring server according to the abnormal state information, and inputting in the monitoring server the aid measure information by a monitor according to the abnormal state information received by the monitoring server.

As one implementation, the abnormal state information can be a difference between the physical state information of the monitored subject and the upper boundary of the preset state information range or the lower boundary of the preset state information range. For example, the physical state information of the monitored subject exceeds the upper boundary of the preset state information range; in this situation, the abnormal state information is the difference between the physical state information and the upper boundary. The abnormal state information can also be the current physical state information of the monitored subject. The abnormal state information is sent to the monitoring server, such that the monitor can acquire the physical abnormal state information of the monitored subject from the monitoring server and determine the further aid measures according to the physical state. After the monitor determines the further aid measures according to the abnormal state information, aid measure information corresponding to the abnormal state information will be input through input devices (e.g., keyboard or touch screen) of the monitoring server, and the aid measure information will be sent to the health monitoring garment through the monitoring server. For example, when the abnormal state information indicates that the blood glucose concentration is too low, the monitor can input aid measure information of injecting glucose in the monitoring server and then the aid measure information will be sent to the health monitoring garment by the monitoring server. Thereafter, a part of the monitored subject that needs injection and how to conduct injection will be indicated by the flexible display screen. In this way, people around the monitored subject can aid the monitored subject timely and accurately.

The method further includes the following after sending the abnormal state information to the monitoring server.

A waiting time is recorded after sending the abnormal state information to the monitoring server.

Preset aid measure information in a memory is read according to the abnormal state information when the waiting time exceeds a preset time threshold value.

The health monitoring garment further includes the memory, where the memory is configured to store all the abnormal state information and multiple preset aid measure information corresponding to multiple abnormal state information. In order to avoid the problem that the aid measure information cannot be received in time due to that the network connection of the monitoring server is unstable or the monitor is not currently attended, the waiting time, between sending the abnormal state information to the monitoring server and receiving the aid measure information from the monitoring server, will be recorded. When the waiting time exceeds the preset time threshold value, it indicates that the current network connection is not stable or no monitor is watching the monitoring server. In this case, by reading the preset aid measure information in the memory according to the abnormal state information, it is possible to avoid delaying the aid on the monitored subject due to failure to timely receive the aid measure information from the monitoring server.

When the network communication of the monitoring server is abnormal and the monitored subject needs the aid, the method further includes the following.

At S152, abnormal state information corresponding to the physical state information is generated when the physical state information does not fall within the preset state information range.

At S154, the preset aid measure information in the memory is read according to the abnormal state information.

It should be understood that, the abnormal state information refers to the physical state information that does not fall within the preset state information range. For example, the abnormal state information can indicate that the blood pressure is too high or too low, the blood glucose concentration is too low, or the like.

The preset aid measure information in the memory is read according to the abnormal state information as follows.

The abnormal state information is compared with at least one of preset abnormal information in the memory.

Preset abnormal information that matches the abnormal state information is obtained from the multiple preset abnormal information.

The preset aid measure information in the memory is read according to the preset abnormal information that matches the abnormal state information.

With aid of common abnormal state information and the preset aid measure information in the memory corresponding to the preset abnormal state information, when the network communication of the monitoring server is abnormal, the abnormal state information is compared with the multiple preset abnormal information in the memory to obtain the preset abnormal information that matches the abnormal state information, thereafter, the preset aid measure information in the memory is read according to the preset abnormal information and the part that needs to be aided of the monitored subject and the aid measure information are indicated by the flexible display screen. In this way, people around the monitored subject can aid the monitored subject timely and accurately.

The part that needs to be aided of the monitored subject and the aid measure information are indicated in a voice broadcast manner after obtaining the aid measure information according to the physical state information.

As one implementation, the health monitoring garment may further include a speaker. When information of the part that needs to be aided of the monitored subject and the aid measure is displayed on the flexible display screen, the information of the part that needs to be aided of the monitored subject and the aid measure can also be indicated by the way of the voice broadcast.

For example, in the case that the abnormal state information indicates that the electrocardiogram is abnormal, the monitor can send information of a part that needs to be pressed and detailed press operations to the health monitoring garment through the monitoring server. And then, the part that needs to be pressed and the detailed press operations are displayed on the flexible display screen of the health monitoring garment. If the monitored subject needs to be aided by beating the chest area, a location of the chest of the monitored subject that needs to be beaten is displayed on the display screen of the health monitoring garment and a fist-shaped pattern can be displayed at the location. In addition, the on-site rescuer can be prompted to beat the location by an assisted text prompt through the flexible display screen or an assisted voice prompt through the speaker. For another example, in the case that the monitored subject needs to be aided by external chest compression, the monitor can send information of a part that needs to be compressed and detailed compress operations to the health monitoring garment through the monitoring server. After that, the part that needs to be compressed and the detailed compress operations are displayed on the flexible display screen of the health monitoring garment and a palm pattern can be displayed at the part (e.g., middle and proximal 1/3 part of the sternum) that needs to be compressed, to prompt the rescuer to perform palm press on the part. Meanwhile, the palm pattern may also be controlled to flash at a certain frequency (for example, 100 times / minute). Furthermore, the rescuer is prompted to press according to the flash frequency by an assisted text prompt through the flexible display screen or an assisted voice prompt through the speaker.

Moreover, for some special conditions, it is necessary to relieve the onset of these conditions or reduce the suffering of the monitored subject by pressing a special acupuncture point on the body. However, most acupuncture points in the body are only small areas. The monitored subject can be properly rescued only when accurate acupuncture points are found. Therefore, when the monitor receives the physical abnormal state information from the health monitoring garment, in the case that the aid measure corresponding to the physical abnormal state information indicates that the monitored subject needs to be aided by pressing a certain acupuncture point of the monitored subject, the monitor can send the specific location of the acupuncture point to the health monitoring garment through the monitoring server. After that, the location of the acupuncture point on the monitored subject is accurately displayed on the flexible display screen of the health monitoring garment to help the rescuer identify the location of the acupuncture point accurately and visually. Moreover, the rescuer is prompted by an assisted text prompt through the flexible display screen or an assisted voice prompt through the speaker. For example, in the case that the physical abnormal state information indicates that the arterial blood pressure is abnormal, it means that the monitored subject may have angina pectoris. In this situation, the monitor can send the location of the Jiquan acupuncture point (an acupuncture point located at the apex of the armpit where the axillary artery pulsates) to the health monitoring garment through the monitoring server. After that, the specific location of the Jiquan acupuncture point on the monitored subject is displayed on the display screen of the health monitoring. Moreover, the rescuer is prompted to aid the monitored subject by pressing the Jiquan acupuncture point by an assisted text prompt through the flexible display screen or an assisted voice prompt through the speaker.

It is to be understood that, in the case that the network communication of the monitoring server is abnormal, the preset aid measure information in the memory can also be displayed on the body of the monitored subject via the flexible display screen, where the aid measure information is read by the health monitoring garment according to the abnormal state information.

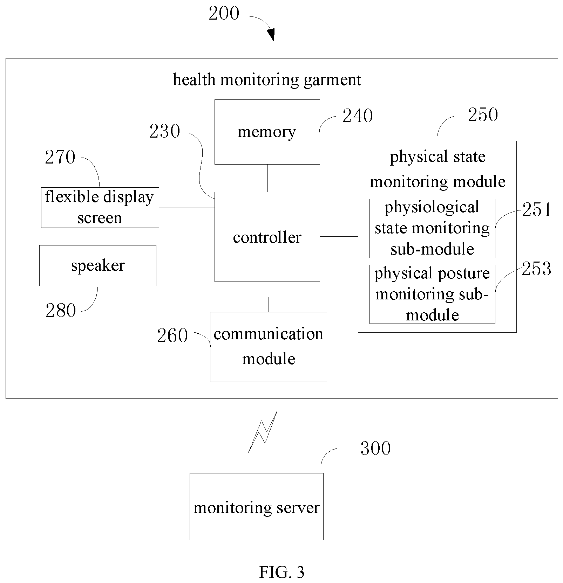

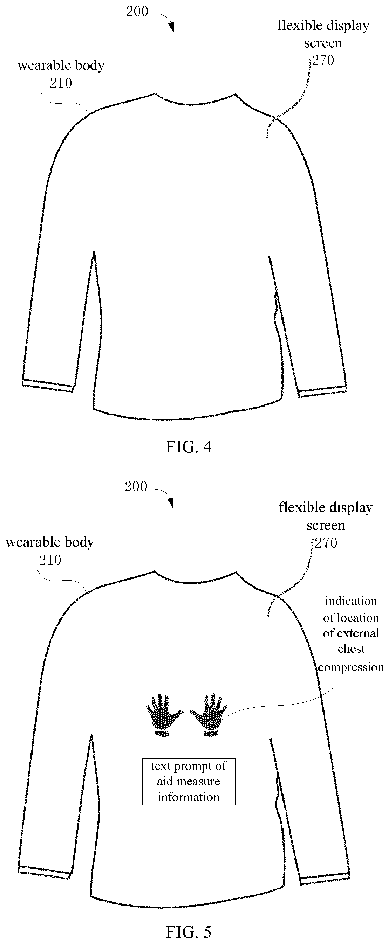

Referring to FIG. 3 and FIG. 4, in one implementation, a health monitoring garment 200 is provided. The health monitoring garment 200 includes a wearable body 210, a controller 230 disposed on the wearable body 210, a physical state monitoring module 250, and a flexible display screen 270.

The controller 230 is electrically connected with the physical state monitoring module 250 and the flexible display screen 270.

The physical state monitoring module 250 is configured to obtain physical state information of a monitored subject and to send the physical state information to the controller 230.

The controller 230 is configured to receive the physical state information, determine whether the monitored subject needs an aid according to the physical state information, and to obtain aid measure information corresponding to the physical state information according to the physical state information based on a determination that the monitored subject needs the aid.

The flexible display screen 270 is configured to indicate a part that needs to be aided of the monitored subject and the aid measure information.

In this embodiment, the wearable body 210 is in a garment-like appearance, which can be worn on the monitored subject and is in elastic contact with the monitored subject. The flexible display screen 270 covers an outer surface of the wearable body 210. When the wearable body 210 is worn on the monitored subject, the flexible display screen 270 can accurately indicate any part covered by the health monitoring garment 200 of the monitored subject.

The health monitoring garment 200 further includes a memory 240 electrically connected with the controller 230, where the memory 240 is configured to store a preset state information range and multiple preset aid measure information.

The controller 230 configured to determine whether the monitored subject needs the aid according to the physical state information is configured to: compare the physical state information of the monitored subject with the preset state information range, and determine that the monitored subject does not need the aid when the physical state information falls within the preset state information range, or determine that the monitored subject needs the aid when the physical state information does not fall within the preset state information range.

The health monitoring garment 200 further includes a communication module 260 configured to communicate with a monitoring server 300. The communication module 260 is further configured to detect whether network communication of the monitoring server 300 is normal after the controller 230 determines whether the monitored subject needs the aid.

When the network communication of the monitoring server 300 is normal, the controller 230 configured to obtain the aid measure information according to the physical state information is configured to: generate abnormal state information corresponding to the physical state information when the physical state information does not fall within the preset state information range, control the communication module 260 to send the abnormal state information to the monitoring server 300, and control the communication module 260 to receive the aid measure information from the monitoring server 300. The aid measure information is generated by the monitoring server 300 according to the abnormal state information or the aid measure information is input in the monitoring server 300 by a monitor according to the abnormal state information received by the monitoring server 300.

The memory 240 is further configured to store a preset time threshold value. After the controller 230 controls the communication module 260 to send the abnormal state information to the monitoring server 300, the controller 230 is further configured to: record a waiting time after sending the abnormal state information to the monitoring server 300 and read the preset aid measure information in the memory 240 according to the abnormal state information when the waiting time exceeds the preset time threshold value.

When the network communication of the monitoring server 300 is abnormal, the controller 230 is further configured to: generate abnormal state information corresponding to the physical state information when the physical state information does not fall within the preset state information range and read the preset aid measure information in the memory 240 according to the abnormal state information.

The memory 240 is further configured to store multiple preset abnormal information corresponding to multiple preset aid measure information. The controller 230 configured to read the preset aid measure information in the memory 240 according to the abnormal state information is configured to: compare the abnormal state information with the multiple preset abnormal information in the memory 240, obtain, from the multiple preset abnormal information, preset abnormal information that matches the abnormal state information, and read the preset aid measure information in the memory 240 according to the preset abnormal information that matches the abnormal state information.

The health monitoring garment 200 further includes a speaker 280. The speaker 280 is configured to indicate the part that needs to be aided of the monitored subject and the aid measure information in a voice broadcast manner.

Referring to FIG. 5, FIG. 5 is a schematic diagram illustrating a usage sate of a health monitoring garment according to an embodiment of the present disclosure. As one implementation, in the case that the abnormal state information indicates that the electrocardiogram is abnormal, the monitor sends the aid measure information to the health monitoring garment 200 through the monitoring server 300. In the case that the aid measure information is external chest compression, the monitor can send information of a part that needs to be compressed and detailed compress operations to the health monitoring garment 200 through the monitoring server 300. After that, the part that needs to be compressed and the detailed compress operations are displayed on the flexible display screen of the health monitoring garment and a palm pattern can be displayed at the part that needs to be compressed (e.g., middle and proximal 1/3 part of the sternum). As illustrated in FIG. 5, the palm pattern is configured to prompt the rescuer to perform palm press on the part. Meanwhile, the palm pattern may also be controlled to flash at a certain frequency (for example, 100 times / minute) to prompt the rescuer to press according to the flash frequency. It should be noted that, in addition to displaying the palm pattern via the flexible display screen 270, a text message of the aid measure information corresponding to the abnormal state information (as illustrated in FIG. 5) may be displayed through the flexible display screen 270 and/or the aid measure information may be played through the speaker 280.

The physical state monitoring module 250 includes a physiological state monitoring sub-module 251 and a physical posture monitoring sub-module 253.

The physiological state monitoring sub-module 251 is configured to obtain physiological state information of the monitored subject.

The physical posture monitoring sub-module 253 is configured to obtain physical posture information of the monitored subject.

The physiological state information includes physical temperature information, heart rate information, blood glucose information, blood oxygen information, blood pressure information, electrocardiogram information, and pulse information. The physical posture information includes moving speed information, physical tilt angle information, and physical orientation information.

In this embodiment, the physiological state monitoring sub-module 251 may include a physical temperature sensor, a heart rate sensor, a blood glucose concentration sensor, a blood oxygen concentration sensor, a blood pressure sensor, an electrocardiogram sensor, a pulse sensor, and the like. The physical posture monitoring sub-module 253 may include an acceleration sensor, a movement speed sensor, an angle sensor, and the like. It can be understood that, the sensors of the physiological state monitoring sub-module 251 and the physical posture monitoring sub-module 253 can be set on the wearable body 210 according to monitoring requirements of the physical state information. The sensors can also be tied to the monitored subject by an elastic bandage, for example, the heart rate sensor may be fixed to the chest of the monitored subject through the elastic bandage and the blood pressure sensor may be fixed to the upper arm of the monitored subject through the elastic bandage. It can be understood that, each sensor of the physiological state monitoring sub-module 251 and the physical posture monitoring sub-module 253 can transmit the monitoring data to the controller 230 through wired communication or wireless communication, where the wireless communication may be in the form of Bluetooth, Wi-Fi, and the like.

It should be noted that, for functions and implementations of the modules/devices of the health monitoring garment 200, reference can also be made to the related descriptions in the method-embodiments illustrated in FIG. 1 and FIG. 2. The disclosure will not be detailed herein again.

According to the method for health monitoring, the physical state information of the monitored subject is obtained, and the aid measure information corresponding to the physical state information is obtained according to the physical state information based on a determination that the monitored subject needs the aid, thereafter, the part that needs to be aided of the body of the monitored subject and the aid measure information are indicated via the flexible display screen. When the monitored subject need the aid, the aid measure information can be presented to people around the monitored subject via the flexible display screen and the part that needs to be aided of the monitored subject can be indicated via the flexible display screen, such that people around the monitored subject can conduct emergency aid on the monitored subject according to the aid measure information and the part that needs to be aided, which has high timeliness and reliability.

While the present disclosure has been described in detail above with reference to the exemplary embodiments, the scope of the present disclosure is not limited thereto. As will occur to those skilled in the art, the present disclosure is susceptible to various modifications and changes without departing from the spirit and principle of the present disclosure. Therefore, the scope of the present disclosure should be determined by the scope of the claims.

## Claims

1. A method for health monitoring, comprising:
obtaining physical state information of a monitored subject via a health monitoring garment, wherein the health monitoring garment comprises a flexible display screen;
determining whether the monitored subject needs an aid according to the physical state information;
obtaining aid measure information corresponding to the physical state information according to the physical state information based on a determination that the monitored subject needs the aid; and
indicating the aid measure information via the flexible display screen.

2. The method of claim 1, wherein the determining whether the monitored subject needs an aid according to the physical state information comprises:
comparing the physical state information of the monitored subject with a preset state information range; and
determining that the monitored subject does not need the aid when the physical state information falls within the preset state information range; or
determining that the monitored subject needs the aid when the physical state information does not fall within the preset state information range.

3. The method of claim 2, wherein the method further comprises the following after the determining whether the monitored subject needs an aid and before the obtaining aid measure information corresponding to the physical state information according to the physical state information:
detecting whether network communication of a monitoring server is normal.

4. The method of claim 3, wherein the network communication of the monitoring server is normal, and the obtaining aid measure information corresponding to the physical state information according to the physical state information comprises:
generating abnormal state information corresponding to the physical state information when the physical state information does not fall within the preset state information range;
sending the abnormal state information to the monitoring server; and
receiving the aid measure information from the monitoring server;
wherein the aid measure information is obtained by the operation selected from the group consisting of generating the aid measure information by the monitoring server according to the abnormal state information, and inputting in the monitoring server the aid measure information by a monitor according to the abnormal state information received by the monitoring server.

5. The method of claim 4, wherein the method further comprises the following after the sending the abnormal state information to the monitoring server:
recording a waiting time after sending the abnormal state information to the monitoring server; and
reading preset aid measure information in a memory according to the abnormal state information when the waiting time exceeds a preset time threshold value.

6. The method of claim 2, wherein the method further comprises the following after the determining that the monitored subject needs the aid:
generating abnormal state information corresponding to the physical state information when the physical state information does not fall within the preset state information range; and
reading preset aid measure information in a memory according to the abnormal state information.

7. The method of claim 5 or 6, wherein the reading preset aid measure information in a memory according to the abnormal state information comprises:
comparing the abnormal state information with multiple preset abnormal information in the memory;
obtaining, from the multiple preset abnormal information, preset abnormal information that matches the abnormal state information; and
reading the preset aid measure information in the memory, according to the preset abnormal information that matches the abnormal state information.

8. The method of any of claims 1 to 6, wherein the method further comprises the following after the obtaining aid measure information corresponding to the physical state information according to the physical state information:
indicating the aid measure information in a voice broadcast manner.

9. The method of any of claims 1 to 6, wherein the physical state information comprises physiological state information and physical posture information of the monitored subject, wherein the physiological state information comprises physical temperature information, heart rate information, blood glucose information, blood oxygen information, blood pressure information, electrocardiogram information, and pulse information, and the physical posture information comprises moving speed information, physical tilt angle information, and physical orientation information.

10. The method of any of claims 1 to 6, wherein the aid measure information comprises a part that needs to be aided displayed on the flexible display screen.

11. The method of any of claims 1 to 6, wherein the aid measure information comprises an aid method displayed on the flexible display screen.

12. A health monitoring garment, comprising a wearable body, a controller disposed on the wearable body, a physical state monitoring module, and a flexible display screen, wherein
the controller is electrically connected with the physical state monitoring module and the flexible display screen;
the physical state monitoring module is configured to obtain physical state information of a monitored subject and to send the physical state information to the controller;
the controller is configured to receive the physical state information, to determine whether the monitored subject needs an aid according to the physical state information, and to obtain aid measure information corresponding to the physical state information according to the physical state information based on a determination that the monitored subject needs the aid; and
the flexible display screen is configured to indicate the aid measure information.

13. The garment of claim 12, further comprising a memory electrically connected with the controller, wherein the memory is configured to store a preset state information range and multiple preset aid measure information; wherein the controller configured to determine whether the monitored subject needs the aid according to the physical state information is configured to:
compare the physical state information of the monitored subject with the preset state information range; and
determine that the monitored subject does not need the aid when the physical state information falls within the preset state information range; or
determine that the monitored subject needs the aid when the physical state information does not fall within the preset state information range.

14. The garment of claim 13, further comprising a communication module configured to communicate with a monitoring server, wherein the communication module is further configured to perform the following after the controller determines whether the monitored subject needs the aid:
detecting whether network communication of the monitoring server is normal.

15. The garment of claim 14, wherein the network communication of the monitoring server is normal, and the controller configured to obtain the aid measure information corresponding to the physical state information according to the physical state information is configured to:
generate abnormal state information corresponding to the physical state information when the physical state information does not fall within the preset state information range;
control the communication module to send the abnormal state information to the monitoring server; and
control the communication module to receive the aid measure information from the monitoring server;
wherein the aid measure information is obtained by the operation selected from the group consisting of generating the aid measure information by the monitoring server according to the abnormal state information, and inputting in the monitoring server the aid measure information by a monitor according to the abnormal state information received by the monitoring server.

16. The garment of claim 15, wherein the memory is further configured to store a preset time threshold value; the controller being further configured to perform the following after the controller controls the communication module to send the abnormal state information to the monitoring server:
recording a waiting time after sending the abnormal state information to the monitoring server; and
reading the preset aid measure information in the memory according to the abnormal state information when the waiting time exceeds the preset time threshold value.

17. The garment of claim 13, wherein the controller is further configured to:
generate abnormal state information corresponding to the physical state information when the physical state information does not fall within the preset state information range; and
read the preset aid measure information in the memory according to the abnormal state information.

18. The garment of claim 16 or 17, wherein the memory is further configured to store multiple preset abnormal information corresponding to multiple preset aid measure information; wherein the controller configured to read the preset aid measure information in the memory according to the abnormal state information is configured to:
compare the abnormal state information with the multiple preset abnormal information in the memory;
obtain, from the multiple preset abnormal information, preset abnormal information that matches the abnormal state information; and
read the preset aid measure information in the memory, according to the preset abnormal information that matches the abnormal state information.

19. The garment of any of claims 12 to 17, wherein the aid measure information comprises a part that needs to be aided and an aid method displayed on the flexible display screen.

20. The garment of any of claims 12 to 17, wherein the physical state monitoring module comprises:
a physiological state monitoring sub-module, configured to obtain physiological state information of the monitored subject; and
a physical posture monitoring sub-module, configured to obtain physical posture information of the monitored subject;
wherein the physiological state information comprises physical temperature information, heart rate information, blood glucose information, blood oxygen information, blood pressure information, electrocardiogram information, and pulse information, and the physical posture information comprises moving speed information, physical tilt angle information, and physical orientation information.
